Europäisches Patentamt

European Patent Office

Office européen des brevets

(11) Veröffentlichungsnummer: **0 364 876**
**A1**

## (12) EUROPÄISCHE PATENTANMELDUNG

(21) Anmeldenummer: 89118925.0

(22) Anmeldetag: 12.10.89

(51) Int. Cl.⁵: **C07C 315/00 , C07C 315/06 , C07C 317/22**

(30) Priorität: 19.10.88 DE 3835527

(43) Veröffentlichungstag der Anmeldung:
25.04.90 Patentblatt 90/17

(84) Benannte Vertragsstaaten:
CH DE FR GB IT LI

(71) Anmelder: BASF Aktiengesellschaft
Carl-Bosch-Strasse 38
D-6700 Ludwigshafen(DE)

(72) Erfinder: Stumpp, Michael, Dr.
An der Marlach 13
D-6705 Deidesheim(DE)
Erfinder: Neumann, Peter, Dr.
Postfach 28
D-6800 Mannheim 31(DE)
Erfinder: Ruehenbeck, Wolfgang, Dr.
Im Bettenklingen 17
D-6943 Birkenau(DE)
Erfinder: Bergner, Michael
Seidelstrasse 1
D-6710 Frankenthal(DE)

(54) Verfahren zur Herstellung von Bis(4-hydroxyphenyl)sulfon.

(57) Verfahren zur Herstellung von Bis(4-hydroxyphenyl)sulfon durch Reaktion von Phenol mit Schwefelsäure in Gegenwart eines Lösungsmittels bei einer Temperatur von 130 bis 220°C und gegebenenfalls in Gegenwart eines Sulfonierungshilfsmittels, wobei man das gebildete Bis(4-hydroxyphenyl)sulfon während oder nach der Reaktion selektiv an einer keimbildenden Oberfläche abscheidet und dem Reaktionsmedium entzieht.

EP 0 364 876 A1

## Verfahren zur Herstellung von Bis(4-hydroxyphenyl)sulfon

Die vorliegende Erfindung betrifft ein neues Verfahren zur Herstellung von Bis(4-hydroxyphenyl)sulfon hoher Reinheit durch Reaktion von Phenol mit Schwefelsäure in Gegenwart eines Lösungsmittels und gegebenenfalls in Gegenwart eines Sulfonierungshilfsmittels, wobei man Bis(4-hydroxyphenyl)sulfon während oder nach der Reaktion an einer Oberfläche abscheidet und dem Reaktionsmedium entzieht.

Bis(4-hydroxyphenyl)sulfon ist für die Herstellung von Fasern, Harzen und hochtemperaturbeständigen Kunststoffen (z.B. Polyethersulfone) von großem wirtschaftlichem Interesse. Da die Eigenschaften der aus Bis(4-hydroxyphenyl)sulfon hergestellten Polymeren außerordentlich stark vom Reinheitsgrad der Monomeren abhängen, sind selektive Syntheseverfahren für Bis(4-hydroxyphenyl)sulfon gefragt.

Bis(4-hydroxyphenyl)sulfon kann durch Reaktion von Phenol mit Schwefelsäure ohne Verwendung eines Lösungsmittels hergestellt werden. Diese Verfahren liefern ein Rohprodukt, das neben 60 bis 70 % Bis(4-hydroxyphenyl)sulfon 20 bis 30 % des Isomeren 2,4'-Dihydroxydiphenylsulfon und ca. 10 % des "Trimeren" 6-Hydroxy[1,3-bis(4-hydroxyphenylsulfonyl]benzol enthält. Alle diese Verfahren erfordern einen hohen Aufwand um Bis(4-hydroxyphenyl)sulfon in hoher Reinheit aus diesem Gemisch zu isolieren.

Durch Verwendung eines Lösungsmittels kann sowohl die Ausbeute, als auch die Selektivität der Reaktion verbessert werden, wie die Beispiele in der JP-A-106 938/1975 oder in der US-A-4 162 270 zeigen.

Gemäß der DE-A-2 708 388 entsteht bei der Reaktion von Phenol mit Schwefelsäure bei Vorhandensein eines Lösungsmittels, z.B. eines aromatischen Kohlenwasserstoffs, Bis(4-hydroxyphenyl)sulfon und das isomere 2,4'-Dihydroxydiphenylsulfon in einem Verhältnis von ca. 3:1. Darüber hinaus entsteht, wie eigene Versuche ergaben, ebenfalls in geringem Maße 6-Hydroxy[1,3-bis(4-hydroxyphenylsulfonyl)]benzol. Die Ausbeute und die Reinheit des Zielproduktes wird dadurch erhöht, daß das Lösungsmittel durch Destillation kontinuierlich aus der Reaktion entfernt oder nach im wesentlichen beendeter Umsetzung abdestilliert wird, wobei es wichtig ist, daß die Destillation im Temperaturbereich von 160 bis 200 °C vorgenommen wird, da nur dann eine Isomerisierung des 2,4'-Isomeren zu Bis(4-hydroxyphenyl)sulfon erreicht wird.

Schließlich sollen nach der Lehre der EP-A-220 004 Phenol und Schwefelsäure in einem inerten Lösungsmittel bei einer Temperatur von 160 bis 200 °C umgesetzt werden, wobei man Bis(4-hydroxyphenyl)sulfon durch selektive Kristallisation aus dem Reaktionsmedium entfernt. Dazu ist es aber notwendig, die Menge des inerten Lösungsmittels genau vorzugeben, um während des gesamten Reaktionsablaufs zu gewährleisten, daß das Reaktionsmedium immer gesättigt ist an Bis(4-hydroxyphenyl)sulfon und immer ungesättigt bleibt an 2,4'-Dihydroxydiphenylsulfon.

Aufgabe der vorliegenden Erfindung war es, ein selektives Verfahren zur Herstellung von Bis(4-hydroxyphenyl)sulfon hoher Reinheit auf der Basis von Phenol und Schwefelsäure zu entwickeln. Das Produkt sollte dabei in einer Reinheit von mehr als 99 % anfallen, ohne daß aufwendige Trennungs- oder Reinigungsstufen erforderlich sind.

Es wurde nun gefunden, daß die Herstellung von Bis(4-hydroxyphenyl)sulfon durch Reaktion von Phenol mit Schwefelsäure in Gegenwart eines Lösungsmittels bei einer Temperatur von 130 bis 220 °C und gegebenenfalls in Gegenwart eines Sulfonierungshilfsmittels vorteilhaft gelingt, wenn man das gebildete Bis-(4-hydroxyphenyl)sulfon während oder nach der Reaktion selektiv an einer keimbildenden Oberfläche abscheidet und dem Reaktionsmedium entzieht.

Bei der erfindungsgemäßen Arbeitsweise ist es vorteilhaft, die Schwefelsäure nicht, wie nach dem Stand der Technik üblich, zusammen mit dem Phenol vorzulegen und zu erhitzen, sondern dem Reaktionsgemisch aus Phenol, Lösungsmittel und gegebenenfalls Sulfonierungshilfsmittel kontinuierlich oder portionsweise im Verlauf der Reaktion, insbesondere während oder nach der Aufheizphase, zuzudosieren. Damit ist es möglich, die Reaktion so zu steuern, daß die unerwünschte Bildung von 6-Hydroxy[1,3-bis(4-hydroxyphenylsulfonyl)]benzol weitgehend zurückgedrängt werden kann. Außerdem kann die selektive Kristallisation des Bis(4-hydroxyphenyl)sulfons gut kontrolliert werden, da zu keiner Zeit ein zweiphasiges Flüssig-Flüssig-System entsteht.

Als Lösungsmittel kommen entweder überschüssiges Phenol oder inerte Lösungsmittel, z.B. aliphatische, cycloaliphatische, araliphatische oder aromatische Kohlenwasserstoffe, wie Benzol, Toluol, Xylol, Ethylbenzol, Diethylbenzol, Decalin oder Tetralin, halogenierte Alkane wie Tetrachlorethan, halogenierte aromatische Kohlenwasserstoffe, wie Chlorbenzol, Dichlorbenzol oder Trichlorbenzol, oder deren Gemische in Betracht.

Bevorzugt sind solche Lösungsmittel, in denen das 2,4'-Isomere besser löslich ist als Bis(4-hydroxyphenyl)sulfon. Beispiele für solche Lösungsmittel, in denen eine große Löslichkeitsdifferenz beider Isomeren vorhanden ist, sind z.B. Chlorbenzol, Dichlorbenzol oder Trichlorbenzol.

Wird überschüssiges Phenol als Lösungsmittel verwendet, so legt man in der Regel einen 0,1- bis 5-fachen Überschuß an Phenol, bezogen auf die Gewichtsmenge an Schwefelsäure, vor und setzt die Schwefelsäure, wie oben beschrieben, zu.

Bei Anwendung von inerten Lösungsmitteln verwendet man im allgemeinen etwa die 0,1- bis 5-fache, vorzugsweise 0,3- bis 3-fache Menge dieser Lösungsmittel, jeweils bezogen auf das Gewicht des eingesetzten Phenols. Größere Mengen sind möglich aber nicht vorteilhaft.

Die zu verwendenden Mengen an Phenol und Schwefelsäure sind nicht besonders kritisch und liegen in dem aus dem Stand der Technik bekannten Bereich. Im allgemeinen werden mindestens 2 Mol Phenol pro Mol Schwefelsäure verwendet.

Die Sulfonierung kann in Gegenwart oder Abwesenheit von Sulfonierungshilfsmitteln, wie Bortrifluorid oder Borsäure, die aus dem Stand der Technik, z.B. Houben-Weyl, Methoden der Organischen Chemie, Band IX, Seite 494 bis 497, 1955, bekannt sind, vorgenommen werden. Dabei sind katalytische Mengen in der Regel ausreichend.

Erfindungsgemäß wird das gebildete Bis(4-hydroxyphenyl)sulfon dem Reaktionsgemisch durch Abscheidung an einer keimbildenden Oberfläche entzogen, wodurch eine Erhöhung der Ausbeute an Bis(4-hydroxyphenyl)sulfon durch Verschiebung des Reaktionsgleichgewichts und eine Produktreinheit von mehr als 99 % resultieren. Das in den obengenannten Lösungsmitteln weitaus besser lösliche, z.B. 50- bis 100-fach besser lösliche, 2,4'-Dihydroxydiphenylsulfon bleibt in der Lösung zurück und scheidet sich nach Isomerisierung bei einer Temperatur, die vorteilhaft oberhalb von 160°C liegt, ebenfalls als Bis(4-hydroxyphenyl)sulfon ab.

Unter einer keimbildenden Oberfläche im erfindungsgemäßen Sinn ist insbesondere eine unterkühlte Oberfläche zu verstehen. Besonders bevorzugt ist eine keimbildende Oberfläche, deren Temperatur etwa 10 bis 50 Grad unterhalb der Reaktionstemperatur liegt. Zur Abscheidung geeignete Oberflächenmaterialien sind z.B. Metall-, Glas-, Keramik- oder temperaturbeständige, inerte Kunststoffoberflächen. Beispielsweise seien Oberflächen aus Stahl, Glas, Emaille oder Teflon genannt.

Weiterhin ist es möglich, die Oberfläche zur besseren Keimbildung aufzurauhen. Dies kann z.B. dadurch geschehen, daß man sie anätzt, anritzt, ankratzt oder schmirgelt.

Durch Animpfen des Reaktionsgemisches mit Fremdkristallen oder vorteilhaft mit reinen Produktkristallen kann die Kristallisation an den beschriebenen Oberflächen weiter gefördert werden.

Die selektive Kristallisation kann vorteilhaft nach zwei prinzipiell verschiedenen Arbeitsweisen vorgenommen werden. Zum einen kann das voranstehend beschriebene Kristallisationsmedium in situ im Reaktionsgemisch vorliegen, d.h. die Sulfonierung wird direkt in Gegenwart der angerauhten und/oder unterkühlten Oberfläche durchgeführt. Zum anderen kann das Kristallisationsmedium extern plaziert sein, und das Reaktionsgemisch wird im Kreis über dieses Medium, das z.B. in Form einer Kühlschlange oder eines Kratzkühlers vorliegt, geleitet.

Die Entfernung des Zielproduktes kann nach beiden Arbeitsweisen sowohl kontinuierlich als auch diskontinuierlich erfolgen. Beispielsweise läßt sich der Feststoff mechanisch abtrennen oder kann mit Hilfe eines Lösungsmittels abgelöst werden.

Die Durchführung des erfindungsgemäßen Verfahrens kann zweckmäßig folgendermaßen vorgenommen werden: Phenol, gegebenenfalls inertes Lösungsmittel sowie gegebenenfalls Sulfonierungshilfsmittel werden im Reaktionsgefäß vorgelegt und auf eine Temperatur von 130 bis 220°C, vorzugsweise 160 bis 200°C, erwärmt. Während der Aufheizphase oder danach wird konzentrierte Schwefelsäure zugetropft, wobei das entstehende Wasser in an sich bekannter Weise ausgekreist und das Lösungsmittel kontinuierlich in das Reaktionsgemisch rückgeführt wird.

Während der Reaktion scheidet sich bis-(4-hydroxyphenyl)sulfon selektiv am externen oder internen Kristallisationsmedium ab. Nach Beendigung der Umsetzung kann das Reaktionsgemisch, vorteilhaft bei einer Temperatur oberhalb von 160°C, nachgerührt werden, um eine vollständige Isomerisierung des in Lösung befindlichen 2,4'-Dihydroxybiphenylsulfons zu bewirken. Die Abtrennung des Feststoffs aus dem Reaktionsgemisch erfolgt in an sich bekannter Weise, so daß sich weitere Ausführungen hierzu erübrigen.

Zwecks weiterer Reinigung, was häufig entbehrlich ist, kann das Produkt in wäßriger Alkalilösung aufgenommen und gegebenenfalls nach Behandlung mit Aktivkohle und Filtration mit Säuren, z.B. Mineralsäuren, insbesondere Schwefelsäure, bei einem pH-Wert von ca. 6 Bis 7 wieder ausgefällt werden.

Bei kontinuierlicher Arbeitsweise wird zweckmäßig wie oben ausgeführt vorgegangen, jedoch werden Phenol und Schwefelsäure im Molverhältnis 2:1 in dem Maße dem Reaktionsmedium zugesetzt, wie sie zu Bis(4-hydroxyphenyl)sulfon abreagieren. Die Abtrennung des Zielprodukts erfolgt hier natürlich ebenfalls in kontinuierlicher Arbeitsweise.

Das nach dem erfindungsgemäßen Verfahren hergestellte Bis(4-hydroxyphenyl)sulfon weist eine Reinheit von mehr als 99 % auf.

Die folgenden Beispiele sollen die Erfindung näher erläutern.

Beispiel 1

In einem 2 l-Planschliffgefäß, das mit einer Glaskühlschlange versehen war, wurden unter Stickstoff 385 g (4,1 Mol) Phenol, 150 ml Chlorbenzol und 60 ml o-Dichlorbenzol vorgelegt und unter Rückfluß erhitzt. Dazu wurden innerhalb von 2 Stunden 209 g (2,05 Mol) 96 gew.%ige Schwefelsäure getropft. Während der gesamten Reaktionszeit wurde Wasser ausgekreist und die organische Phase in das Reaktionsgefäß zurückgeführt. Anschließend wurde die Temperatur der Kühlschlange auf ca. 135° C abgekühlt, während die Temperatur des Reaktionsmediums ca. 168° C betrug, woraufhin sich Bis(4-hydroxyphenyl)sulfon an der Kühlschlange abzuscheiden begann. Man rührte noch 1,5 Stunden bei 168° C nach, dann wurde die Kühlschlange entfernt, der gebildete Niederschlag in wäßriger Alkalilösung aufgenommen, mit Aktivkohle behandelt, filtriert und mit Schwefelsäure bei pH = 6,5 wieder ausgefällt. Nach Trocknen erhielt man Bis(4-hydroxyphenyl)sulfon in einer Reinheit von > 99,5 (HPLC) und in einer Ausbeute von 70 %, bezogen auf eingesetzte Schwefelsäure.

Beispiel 2

In einem 2 l-Planschliffgefäß, das im Kreislauf mit einem Kratzkühler geschaltet war, wurden unter Stickstoff 385 g (4,1 Mol) Phenol, 150 ml Chlorbenzol und 60 ml o-Dichlorbenzol vorgelegt und unter Rückfluß erhitzt. Dazu wurden innerhalb von 2 Stunden 209 g (2,05 Mol) 96 gew.%ige Schwefelsäure getropft. Während der gesamten Reaktionszeit wurde Wasser ausgekreist und die organische Phase in das Reaktionsgefäß zurückgeführt. Anschließend wurde die gesamte Reaktionslösung (Temperatur: 168° C) in einem geschlossenen, beheizten System über den Kratzkühler (Temperatur: 100° C) gepumpt und in das Reaktionsgefäß zurückgeführt. Das am unterkühlten Kühler abgeschiedene Produkt wurde kontinuierlich abgekratzt und entfernt. Nach 3 Stunden Reaktionszeit erhielt man Bis(4-hydroxyphenyl)sulfon in einer Reinheit von > 99,5 % (HPLC) und in 90 % Ausbeute. Zur weiteren Reinigung kann wie in Beispiel 1 beschrieben verfahren werden.

Beispiel 3

In einem 2 l-Planschliffgefäß, das im Kreislauf mit einem Kratzkühler geschaltet war, wurden unter Stickstoff 385 g (4,1 Mol) Phenol, 150 ml Chlorbenzol und 60 ml o-Dichlorbenzol vorgelegt und unter Rückfluß erhitzt. Dazu wurden innerhalb von 2 Stunden 209 g (2,05 Mol) 96 gew.%ige Schwefelsäure getropft. Während der gesamten Reaktionszeit wurde Wasser ausgekreist und die organische Phase in das Reaktionsmedium zurückgeführt. Anschließend wurde die gesamte Reaktionslösung (Temperatur: 168° C) in einem geschlossenen beheizten System über den Kratzkühler (Temperatur: 100° C) gepumpt und über ein kontinuierlich entleerbares Filteraggregat in das Reaktionsmedium zurückgepumpt. Das im Filteraggregat zurückgehaltene Bis(4-hydroxyphenyl)sulfon wurde kontinuierlich ausgetragen. Zur Aufrechterhaltung des Stoffstromes wurden dem Reaktionsmedium soviel an Phenol und Schwefelsäure zugesetzt, wie es dem Austrag an Bis(4-hydroxyphenyl)sulfon entsprach. Nach 10 Stunden Reaktionszeit wurden insgesamt 1650 g Bis(4-hydroxyphenyl)sulfon erhalten (Ausbeute: 89 %).
Die folgende Tabelle verdeutlicht den Ablauf der Umsetzung.

| Reaktionszeit | Einsatzstoffe | | Zielprodukt |
|---|---|---|---|
| | Schwefelsäure | Phenol | |
| 3 h | 209 g (2,05 Mol) | 365 g (4,1 Mol) | 450 g (1,8 Mol) |
| 4 h | 306 g (3,0 Mol) | 534 g (6 Mol) | 650 g (2,6 Mol) |
| 10 h | 754 g (7,4 Mol) | 1317 g (14,8 Mol) | 1650 g (6,6 Mol) |

**Ansprüche**

1. Verfahren zur Herstellung von Bis(4-hydroxyphenyl)sulfon durch Reaktion von Phenol mit Schwefelsäure in Gegenwart eines Lösungsmittels bei einer Temperatur von 130 bis 220°C und gegebenenfalls in Gegenwart eines Sulfonierungshilfsmittels, dadurch gekennzeichnet, daß man das gebildete Bis(4-hydroxyphenyl)sulfon während oder nach der Reaktion selektiv an einer keimbildenden Oberfläche abscheidet und dem Reaktionsmedium entzieht.

2. Verfahren gemäß Anspruch 1, dadurch gekennzeichnet, daß man als Lösungsmittel aliphatische, cycloaliphatische, aromatische oder araliphatische Kohlenwasserstoffe oder Kohlenwasserstoffgemische verwendet.

3. Verfahren gemäß Anspruch 1, dadurch gekennzeichnet, daß man die Abscheidung des Bis(4-hydroxyphenyl)sulfon an einer keimbildenden, unterkühlten Oberfläche vornimmt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,D | EP-A-0 220 004 (AMOCO CORP.) <br> * Seiten 4-6, Beispiele 1,2; Ansprüche * | 1-3 | C 07 C 315/00 <br> C 07 C 315/06 <br> C 07 C 317/22 |
| X | FR-A-1 276 779 (SHELL) <br> * Seite 2, Spalte 2, Linien 1-58; Seite 3, Spalte 1, Linien 1-14; Ansprüche * | 1-3 | |
| X | US-A-3 297 766 (K.B. BRADLEY et al.) <br> * Seite 1, Beispiel 1; Seite 2 * | 1-3 | |
| X | GB-A-2 030 566 (ICI) <br> * Seite 1, Beispiel 1 * | 1-3 | |
| X | DE-A-3 141 455 (TOKAI DENKA KOGYO K.K.) <br> * Seite 12, Beispiel 3; Seite 13 * | 1-3 | |
| A,P | EP-A-0 293 037 (AKZO N.V.) <br> * Zusammenfassung; Seite 4, Beispiel 1 * | 1-3 | RECHERCHIERTE SACHGEBIETE (Int. Cl.5) |
| A | DE-B-1 618 023 (WITCO CHEMICAL CO.) <br> * Spalten 3,4 * | 1-3 | C 07 C 315/00 <br> C 07 C 317/00 |

**Der vorliegende Recherchenbericht wurde für alle Patentansprüche erstellt**

| Recherchenort | Abschlußdatum der Recherche | Prüfer |
|---|---|---|
| BERLIN | 28-11-1989 | RUFET J.M.A. |

EPO FORM 1503 03.82 (P0403)

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument